# EUROPEAN PATENT APPLICATION

(11) **EP 2 620 462 A1**
(43) Date of publication of application: **31.07.2013**
(21) Application number: 12425013.5
(22) Date of filing: 24.01.2012
(51) Int. Cl.: C08G 63/78, C12P 7/62

(54) **Process for the production of polyesters through synthesis catalyzed by enzyme**

(71) Applicant: AMBIENTE E NUTRIZIONE S.r.l., 20089 Rozzano (MI) (IT); Sprin S.p.A., 34100 Trieste (IT)
(72) Inventor: Cerea, Giuseppina, 20089 Rozzano, Milano (IT); Gardossi, Lucia, 34100 Trieste (IT); Sinigoi, Loris, 34100 Trieste (IT); Fattor, Diana, 34100 Trieste (IT)
(74) Representative: Zambardino, Umberto

(57) **Abstract**

The present invention relates to a new process for the production of polyesters through polymerization catalyzed by enzyme of at least one monomer characterized in that it comprises the step of running a mixture comprising said at least one monomer and said enzyme in the form of a thin, turbulent and dynamic layer in contact with a wall kept at a predetermined temperature. The process is conveniently carried out in a turbo-reactor (A) comprising a tubular cylindrical body (1), provided with a heating or cooling jacket (4), closed at the opposite ends by bottoms (3, 4), provided with at least one input opening (5, 6) and at least one output opening (7, 11), and inside which a coaxial bladed rotor (8) is rotably supported, and optionally also in a further turbo-reactor (B) essentially identical to the previous one.

## Description

### Field of application

The present invention relates to a process for the production of polyesters through synthesis catalyzed by enzyme.

### Known art

As known, polyesters represent important industrial products since they find different applications in particular in the production of moulded items for use, for example, in the automotive field, in the production of adhesives and as coating films. They also represent intermediates for the production of polyurethanes, which are usually used in the manufacture of moulded items as well.

Typically, polyesters are produced by chemical synthesis through processes involving high-temperature polymerization or polycondensation of respective monomers in the presence of a suitable catalyst, for relatively long times. The competition between esterification, transesterification and hydrolysis reactions limits the average molecular weight of the final products. Furthermore, said processes involve the formation of more or less important amounts of undesirable byproducts and have the drawback of using "toxic" catalysts (usually organo-metals), which can also be difficult to remove.

In the light of said drawbacks, in the last decades new processes for the production of polymers, and in particular of polyesters, were studied, wherein the synthesis is catalyzed by suitable biocatalysts, in particular by enzymes.

In fact, the use of enzymes as biocatalysts for synthesis processes, in principle, provides undoubted advantages mainly in terms of efficiency of the reaction, which can be carried out in mild conditions, of lower environmental impact and of high enantiomeric selectivity and/or region-selectivity that can also lead to the achievement of new products not obtainable or hardly obtainable through conventional synthesis methods.

In the case of the synthesis of polyesters, different processes that use an enzyme, in particular a lipase, as a biocatalyst, for the whole duration of the polymerization or only for some parts of it, were conceived and set up.

For example, Patent Application WO 94/12652 by Baxenden discloses a process for the production of polyesters having certain repeating units as defined in such application, wherein the monomers corresponding to said repeating units are polymerized in the absence of solvent and in the presence of a lipase as a biocatalyst. In particular, as a biocatalyst, a lipase deriving from *Candida antarctica* (CALB) is used, in its free form or immobilized on a polymeric support. Polymerization can occur in a single step or in two distinct steps. In the latter case, in the first step, also called oligomerization step, the starting monomers are converted into oligomers in the presence of the biocatalyst, after which the biocatalyst is removed, for example by filtration, and the polymerization is completed in the absence of biocatalyst in a second step.

The application WO 98/55642 by Baxenden discloses a process for the production of polyesters similar to that forming the subject-matter of the abovementioned application WO 94/12652 and in which it is proposed, inter alia, to recover the immobilized enzyme from the reaction medium and to recycle it for a subsequent polymerization cycle.

However, in the procedures described above, a significant part of the enzyme is lost in the reaction medium due to its detachment from the polymeric support thereby rendering the whole process (and in particular the operation of recycling the immobilized enzyme separated from the reaction medium) not very efficient and expensive, at least for an industrial application. To increase the efficiency of the process, in the application WO 98/55642 it is suggested to add a portion of "fresh" enzyme to the recycled immobilized enzyme (page 12, lines 1-5), but this significantly increases the costs to a level that can be unacceptable for an industrial application. The above problems are also confirmed in the subsequent publication F. Binns; P. Harffey; S.M. Roberts; A. Taylor; J. Chem. Soc. Perkin Trans 1, 1999, 2671-2676 that has among the authors the same inventors designated in the patent applications mentioned above. In that paper, it is additionally emphasized that the immobilized enzyme recovered from the reaction medium loses most of its original activity and it is suggested to add amines, namely triethylamine (TEA), to the polymerization medium to recover part of such activity.

More recently, the publication C. Korupp; R. Weberskirch; J.J. Müller; A. Liese; L. Hilterhaus, Organic Process Research & Development 2010, 14, 1118-1124 discloses the results of a "scale up" study related to the enzymatic production of glycerol adipate on a 500 g scale in a heated apparatus and in the absence of solvent. The study is focused on the influence of various reaction parameters (temperature, pressure, enzyme concentration, type and speed of shaking and reaction time) on the yield of conversion and average molecular weight of the final product. In particular, it is shown that it is possible to obtain average molecular weights of the final product of the order of 2000-3000 Da only in case of high conversion yields, which are however achievable only through a prudent choice of the investigated reaction parameters.

In spite of the remarkable efforts made to date, at the state of the art most processes for the production of polyesters through synthesis catalyzed by enzymes result not practicable on an industrial scale or at least not economically advantageous.

Among the reasons that block or at least limit the industrial application of said known processes catalyzed by enzymes, the most significant relate to the limited "adaptability" and/or stability of the biocatalyst, even if immobilized, in the more "severe" process conditions typical of an industrial plant, to the relatively high cost of the biocatalyst and to the loss of the catalyst due to the detachment from the polymeric support as previously discussed. Additionally, the reactions of interest are normally characterized by high viscosity of the system that restricts diffusive processes and then determines slow reaction kinetics with consequent low molecular weight of the product. Even if it is possible to carry out the reaction under continuous shaking, it must be said that, beyond a certain limit of viscosity, the shear forces to be applied to keep the product homogeneous would be too high, so that the number average molecular weight (Mn) results to be limited.

It should be additionally noted that, in view of developing low environmental impact and economically more competitive processes, it is of primary interest to avoid the use of organic solvents in the polycondensation process. Hence, diffusive processes should be promoted, both with a suitable formulation of the biocatalysts and with a suitable configuration of the reactors.

The main object of the present invention is then to provide a new process for the production of polyesters through synthesis catalyzed by enzyme having such functional characteristics that it can be implemented in an efficient manner on an industrial level, then overcoming or at least attenuating the drawbacks of the abovementioned known processes.

Further object of the present invention is to provide a new process as above, which allows obtaining polyesters in a simple manner and at relatively low costs compared with those of the traditional processes currently in use on an industrial level.

### Summary of the invention

Such objects are reached, according to the invention, through a process for the production of polyesters through polymerization or polycondensation catalyzed by enzyme of at least one monomer characterized in that it comprises the step of running a mixture comprising said at least one monomer and said enzyme in the form of a thin, turbulent and dynamic layer in contact with a wall kept at a predetermined temperature.

In a preferred embodiment, the process according to the invention comprises the following steps:
- preparing a mixture comprising at least one monomer and an enzyme,
- running at least once said mixture in the form of a thin, turbulent and dynamic layer in contact with a wall kept at a predetermined temperature, obtaining a polymeric product comprising polyesters.

In another preferred embodiment, the process according to the invention comprises the steps of:
- preparing a mixture comprising at least one monomer and an immobilized enzyme,
- running at least once said mixture in the form of a thin, turbulent and dynamic layer in contact with a wall kept at a predetermined temperature, obtaining a mixture comprising oligomers,
- essentially separating said immobilized enzyme from said mixture comprising oligomers,
- running at least once said mixture comprising oligomers and essentially free of said enzyme in the form of a thin, turbulent and dynamic layer in contact with a wall kept at a predetermined temperature, obtaining a polymeric product comprising polyesters.

In the present description, the term "monomer" means any substance among those defined hereunder, which molecules are provided with functional groups able to combine recursively with other identical or reactively complementary molecules to form polyesters.

The term "polyester" or "polyesters" means a polymeric material produced according to the process of the invention deriving from the combination of a high number of molecules of a monomer or of reactively complementary monomer molecules as defined hereunder.

The term "oligomer" or "oligomers" means an intermediate material produced during the actuation of the process of the invention deriving from the combination of a finite and limited number of molecules of a monomer or of reactively complementary monomer molecules as defined hereunder. Oligomers obtainable in the actuation of the process according to the invention can be for example, dimers, trimers, tetramers, pentamers etc. and such terms denote the total number of monomeric units in the oligomer.

The term "thin, turbulent and dynamic layer" in the present description means a layer or a film of material (reaction mixture) having a thickness preferably comprised between 1 mm and 20 mm and moving in a turbulent flow regime in contact with a wall kept at a predetermined temperature (heated or cooled).

The term "immobilized enzyme" means any material in which the enzyme is coupled with a support or in which enzymatic molecules are coupled together to form an essentially solid aggregate in the absence of a support, or an enzymatic aggregate in turn coupled with a support. The coupling can be constituted both of physical interactions, for example through adsorption or ionic bond, and of chemical interactions, preferably through covalent bonds.

In the process according to the invention, the enzyme immobilization is carried out preferably in a covalent way and optionally on an inert support (polymeric support), that is to say through the formation of intermolecular covalent bonds between enzymatic molecules and/or covalent bonds between the enzyme and the support.

The enzyme immobilization in a covalent way can be carried out in different ways among which the following are indicated, by way of indication, and not of limitation:
- immobilization of the enzyme on a support through covalent bonds between respective functional groups of the enzyme and of the support (the latter being optionally "functionalized" beforehand with suitable functional groups),
- immobilization of the enzyme on a support through a bifunctional reagent (for example, glutaraldehyde) able to create a net of covalent bonds (cross-links) between the enzymatic molecules and the support in such a way to keep the enzyme stably adherent to the support,
- formation of enzymatic aggregates through a bifunctional reagent able to create covalent bonds (cross-links) between enzymatic molecules to form a solid form of the biocatalyst in the absence of a solid support.

In the process according to the invention, in the case of the conversion of the mixture comprising at least one predetermined monomer and an enzyme into oligomers, or of the direct conversion of said mixture into the polymeric final product, the wall in contact with the thin, turbulent and dynamic layer of said mixture is kept at a temperature at least of 35°C, preferably between 45°C and 130°C.

Instead, in the case of the conversion of the mixture of oligomers free of immobilized enzyme in the form of a thin, turbulent and dynamic layer into polyesters, the temperature of the wall in contact with such mixture is at least 50°C, preferably between 90°C and 300°C.

Conveniently, the process according to the invention can be carried out with the aid of an apparatus called turbo-reactor, comprising a tubular cylindrical body, provided with a heating jacket, closed at the opposite ends by bottoms, provided with at least one input opening and at least one output opening, and inside which a coaxial bladed rotor is rotably supported.

In such case, the process according to the invention can use a turbo-reactor as above, in which the whole conversion of the starting monomer or monomers into the final product (polyesters) is carried out in the presence of an enzyme, or in which only the conversion of the starting monomer or monomers to oligomers is carried out again in the presence of an enzyme.

In the latter case, the subsequent conversion of the oligomers into polyesters can be carried out, after removal of the enzyme, preferably in a second turbo-reactor as defined above or in a functionally equivalent reactor. By employing a turbo-reactor as above, the process according to the invention can comprise the steps of:
- feeding, through said at least one input opening, a continuous flow of a mixture comprising at least one monomer and an enzyme into said turbo-reactor or feeding, through respective input openings, a continuous flow comprising at least one monomer and a separated continuous flow comprising one enzyme and optionally at least one monomer into said turbo-reactor, so as to form said mixture inside of it, the temperature of the wall of said turbo-reactor being at least 35°C, preferably between 45°C and 130°C,
- subjecting at least once said mixture to the mechanical action of the bladed rotor set in rotation at a speed of at least 150 rpm, with the consequent centrifugation of said mixture against the heated wall and formation of a thin, tubular and dynamic layer wherein said mixture is mechanically kept in a condition of turbulence by the blades of said bladed rotor,
- making said thin, turbulent and dynamic layer move forward, towards said at least one output opening of the turbo-reactor,
- continuously discharging a flow of a mixture comprising polyesters from said at least one output opening of said turbo-reactor,
- optionally separating said enzyme from said mixture comprising polyesters.

Separation of the enzyme from the polymeric final product (mixture of polyesters) is preferable where it can be carried out relatively with ease, for example in the case of employment of an immobilized enzyme with the respective possibility of recycling it, as it will be better explained hereunder. In other cases, however, the enzyme can be left unseparated from the polymeric final product due to, for example, considerations of costs of the enzyme and/or complexity and costs of separation.

By employing two turbo-reactors in sequence as above, the process according to the invention can comprise the steps of:
- feeding, through said at least one input opening, a continuous flow of a mixture comprising at least one monomer and an immobilized enzyme into a first turbo-reactor or feeding, through respective input openings, a continuous flow comprising at least one monomer and a separated continuous flow comprising an immobilized enzyme and optionally at least one monomer into said first turbo-reactor, so as to form said mixture inside of it, the temperature of the wall of said first turbo-reactor being at least 35°C, preferably between 45°C and 130°C,
- subjecting at least once said mixture to the mechanical action of the bladed rotor of said first turbo-reactor set in rotation at a speed of at least 150 rpm, with the consequent centrifugation of said mixture against the heated wall and formation of a thin, turbulent and dynamic layer, wherein said mixture is mechanically kept in a condition of high turbulence by the blades of said bladed rotor,
- making said thin, turbulent and dynamic layer move forward, towards said at least one output opening of said first turbo-reactor,
- continuously discharging a flow of a mixture comprising oligomers from said at least one output opening of said first turbo-reactor,
- separating said immobilized enzyme from said mixture comprising oligomers, obtaining a mixture of oligomers essentially free of said immobilized enzyme,
- feeding, through said at least one input opening, a continuous flow of said mixture of oligomers essentially free of said immobilized enzyme into a second turbo-reactor, which temperature of the wall is at least 50°C, preferably between 90°C and 300°C,
- subjecting said mixture of oligomers essentially free of said immobilized enzyme to the mechanical action of the bladed rotor of said second turbo-reactor set in rotation at a speed of at least 150 rpm, with the consequent centrifugation of said mixture against the heated wall and formation of a thin, turbulent and dynamic layer, wherein said mixture is mechanically kept in a condition of high turbulence by the blades of said bladed rotor,
- making said thin, turbulent and dynamic layer move forward, towards said at least one output opening of said second turbo-reactor,
- continuously discharging a flow of a mixture of polyesters from said at least one output opening of said second turbo-reactor.

Thanks to the formation of a tubular, thin, dynamic, and turbulent layer for example by means of the action of the bladed rotor set in rotation at high speed, and of the controlled temperature of the wall in contact with such layer, it was surprisingly found that optimal conditions of mass and thermal transfer are established, to the advantage of the efficiency and yield of conversion into oligomers or polymers.

More in particular, it was surprisingly found that the mechanical action of splitting and centrifugation of the reaction mixture performed for example by the bladed rotor creates optimal conditions of contact between the biocatalyst (enzyme) and the mass to catalyze, as well as it exerts optimal shear forces on the oligomer or polymer in the making without determining a substantial damage of the biocatalyst due to mechanical action, in particular where the biocatalyst is immobilized on a support, preferably in a covalent way.

Then, the biocatalyst can be recovered at the end of a production cycle and advantageously reused for further production cycles keeping, at the same time, a suitable catalytic efficiency, which is clearly translated into a considerable saving on production costs in particular for an industrial application. This is highlighted in particular in example 2, reported hereunder in the present description.

At the same time, the fact that the thin layer of mixture is made move forward in a turbulent manner and in continuous contact with a part at a constant temperature, advantageously allows to effectively and continuously remove from the reaction mixture the reaction byproducts (mainly water but also, for example, alcohols) in the form of vapors, preventing competitive reactions of hydrolysis, with a consequent maximization of the polycondensation process to form oligomers or polymers.

It should be additionally noted that in the process conditions according to the invention and with the continuous removal of water from the reaction mixture (in the form of steam) there is also the advantageous effect of being able to exploit the latent heat of evaporation of water and the optionally negative enthalpy of the reaction of polycondensation. This allows, advantageously, to keep the temperature, to which the polymeric chain in the making is subjected, at values lower than the predetermined temperature of the wall in contact with such polymeric chain, hence remaining in a safe and continuously manageable range of temperatures.

In particular, depending on the required range of lengths of the polymeric chain of the final product (oligomers or polymers), the expert in the art can properly program and continuously manage residence times and temperatures of the polymeric chain in the making.

In the process according to the invention, the rotation speed of the bladed rotor is preferably comprised between 150 and 700 rpm.

The input flow rate of the mixture comprising at least one monomer and an enzyme into the first turbo-reactor or of the mixture of oligomers free of enzyme into the second turbo-reactor is generally comprised between 50 and 500 kg/h, when the turbo-reactor has a diameter between 350 mm and 420 mm.

The mean residence time of the mixture comprising the at least one monomer and an enzyme or of the mixture of olgomers essentially free of enzyme in the respective turbo-reactor is generally between 5 minutes and 30 minutes and it is selected depending on the chemical reactivity and stability of reagents, biocatalyst, intermediates and/or final product and on the desired average molecular weight of the final product.

Additionally, it is possible to feed into said turbo-reactor or first turbo-reactor, in co-current concomitantly with said flow of the mixture comprising the at least one monomer and an enzyme and through respective input openings, a continuous flow of an inert gas, for example nitrogen, and/or suitable additives in the reaction mixture.

Such additives can comprise, by way of indication and not of limitation, chain modifying agents and/or plasticizers, which addition to the reaction mixture comprising the at least one monomer and the enzyme can have the beneficial effect of:
- preventing or reducing a possible effect of inactivation of the enzyme by the at least one monomer, thereby promoting the enzymatic catalysis and/or
- operating a reaction of "chemical" conversion of the at least one monomer simultaneously with that catalyzed by the enzyme.

It is also possible to add the above additives to the reaction mixture during the constitution of such mixture and i.e. in a mixing step preceding the treatment of the mixture in the reaction conditions of the present invention (thin and turbulent layer made move forward along a wall at a predetermined temperature), a treatment carried out, for example, in a turbo-reactor as above.

The addition of an inert gas, in particular nitrogen, preferably preheated at a temperature between 80°C and 150°C, instead, has the advantage of preventing, if necessary, possible collateral oxidative reactions on the polymeric chains in the making.

Additionally, it could be convenient to allow the release from the turbo-reactor, through said at least one output opening, of vapors of the reaction byproducts, mainly water and/or alcohols, both during the polymerization to form oligomers, and during the (subsequent) polymerization to form the final products. If necessary, such release of vapors can optionally be forced, for example by applying a reduced pressure inside the turbo-reactor, preferably equal to or lower than 700 mmHg (93.3 kPa).

In the case that two turbo-reactors in sequence are used, as defined above, the output flow of the mixture of oligomers from the first turbo-reactor undergoes a separation step of the immobilized enzyme, carried out preferably continuously, before being fed into the second turbo-reactor.

Such separation step can be carried out according to any conventional method and is preferably carried out by filtration or centrifugal separation.

The filtration can be carried out for example forcing the flow of the mixture of oligomers through suitable molecular sieves of a filtration unit placed between the first turbo-reactor and the second turbo-reactor and in fluid-flow communication with them. In the case of a centrifugal separation, instead, the flow of the mixture of oligomers can undergo a continuous centrifugation in a centrifugal extractor (decanter), placed between the first turbo-reactor and the second turbo-reactor and in fluid-flow communication with them as well, recovering the biocatalyst (immobilized enzyme) in a head zone of such centrifugal extractor.

In any case, the separated biocatalyst can be advantageously recovered and reused (recycled) for a further polymerization cycle of the monomer or of the monomers to oligomers or polyesters.

It should be noted that, in some cases, the removal of the biocatalyst after the initial step of conversion of the starting monomer or monomers into oligomers (oligomerization) can be advantageous, to prevent damage of the biocatalyst by mechanical action in the subsequent step of conversion into polyesters. In fact, as the reaction proceeds towards the formation of the final product, the viscosity of the reaction mixture increases, so that always increasing shear forces are required to shake and move the reaction mixture in an efficient manner. Such shear forces, in some cases, might damage the biocatalyst if it was still present in the reaction mixture after the oligomerization step.

In the process according to the invention, polyesters can be obtained by polymerization or polycondensation of one or several monomers as indicated hereunder.

According to an embodiment, polyesters can be obtained through the process according to the invention from monomers selected from at least one hydroxycarboxylic acid or a carboxylic acid derivative thereof (for example, an ester or a lactone) and hence such polyesters comprise repeating units comprised of residues of said monomers.

Hydroxycarboxylic acids suitable for use in the process according to the invention include those having the following formula:

HOCH₂-R¹-CO₂H

wherein R¹ is a bond or a bivalent radical of a preferably C1 to C12 alkyl group, or a bivalent radical of an aromatic group.

According to another embodiment, polyesters can be obtained through the process according to the invention from the combination of molecules of the following monomers: 1) at least one dicarboxylic acid or a carboxylic acid derivative thereof, 2) at least one hydroxylamine, diol, polyol, sugar or a derivative thereof and optionally 3) at least one hydroxycarboxylic acid or a carboxylic acid derivative thereof. Resulting polyesters hence present repeating units comprised of residues of said monomers.

Dicarboxylic acids (or diacids) usable in the present invention should be meant as molecules presenting at least two carboxylic groups. In particular, suitable dicarboxylic acids include those of formula:

HO₂C-R²-CO₂H

wherein R² is a bond or a bivalent radical as defined above for R¹.

Suitable diols include those of formula

HOCH₂-R³-CH₂OH

wherein R³ is a bond or a bivalent radical as defined above for R¹. Suitable polyols include those of formula

HOCH₂-R⁴-CH₂OH

wherein R⁴ is a bivalent radical as defined above for R¹ bearing at least one hydroxyl substituent.

Sugars suitable in the process according to the invention include monosaccharides, disaccharides, trisaccharides and oligosaccharides. Examples of suitable sugars include, but are not limited to, glucose, mannose and fructose (monosaccharides); sucrose, lactose, maltose, trehalose (disaccharides); raffinose (trisaccharide). Particularly preferred are sucrose and fructose.

Sugar derivatives include, but are not limited to, glycosides and amino sugars. Glycosides preferably present an alkyl group having in turn preferably 1 to 8 carbon atoms, for example a methyl glycoside or a butyl glycoside. An example of amino sugar is consisting of glutamine.

Suitable hydroxyamines include those of formula:

R⁵HNCH₂-R⁶-CH₂OH

wherein R⁶ is a bond or a bivalent radical as defined above for R¹, and R⁵ is hydrogen or a C1 to C12 alkyl group.

Each of the groups mentioned above can be substituted or unsubstituted and, in the case of alkyl groups, it can be cyclic, with straight or branched chain optionally having at least one carbon-carbon double bond in cis or trans conformation and optionally having at least one triple carbon-carbon bond. When the alkyl group has more than one double or triple carbon-carbon bonds, such bonds can be conjugated or not conjugated. Each alkyl or aromatic group can be optionally substituted with one or several substituents (which in case of two or more substituents can be the same or different), each selected from halogen atoms, for example, fluorine, chlorine or bromine, hydroxyl, epoxy, -NHR⁷ where R⁷ preferably stands for hydrogen or C1-C12 alkyl, -OR⁸ where R⁸ preferably stands for hydrogen or C1-C12 alkyl, carbonyl, -CO₂R⁹ where R⁹ preferably stands for hydrogen or C1-C12 alkyl, -SR where R preferably stands for hydrogen or a C1-C12 alkyl.

The diol has preferably 2 to 14 carbon atoms and is a α,ω-diol, for example 1,4-butanediol, diethylene glycol, ethylene glycol, propylene glycol, pentanediol, hexane-1,6-diol or dodecane-1,12-diol, most preferred is 1,4-butanediol. Preferably, the diacid has 2 to 14 carbon atoms, for example oxalic acid, succinic acid, fumaric acid, citric acid, malic acid, malonic acid, maleic acid or adipic acid. Particularly preferred is adipic acid.

The hydroxycarboxylic acid preferably has 2 to 14 carbon atoms, for example glycolic acid, lactic acid, 2-hydroxy-butyric acid, 2-hydroxy-isobutyric acid, 2-hydroxy-caproic acid, 2-hydroxy-isocaproic acid, citric acid or malic acid.

Suitable aromatic dicarboxylic acids include, but not limited to, phthalic acid (1,2-benzenedicarboxylic acid), isophthalic acid (1,3-benzenedicarboxylic acid) and terephthalic acid (1,4-benzenedicarboxylic acid).

Examples of suitable aminocarboxylic acids include, but are not limited to, 4-amino-butyric acid, 6-amino-caproic acid, 12-aminododecanoic or 7-amino-heptanoic acid. In the present description, the term "carboxylic acid derivative" refers to esters, amides and anhydrides of acids, lactones and nitriles. An ester of a diacid can be a monoester or a diester, for example a monoalkyl or dialkyl ester. Preferably alkyl groups have 1 to 4 carbon atoms each and the most preferred derivative is a methyl or ethyl ester or diester, the most preferred being methyl adipate and dimethyl adipate.

Polyols have at least three hydroxyl groups, at least two of which are preferably primary or secondary hydroxyl groups without steric hindrance. Suitable polyols include pentaerythritol and triols, in particular glycerol, as well as sugar alcohols containing 5 to 12 carbon atoms, for example sorbitol and mannitol and derivatives of such sugar alcohols for example isosorbide (product of the double dehydration of sorbitol).

Polyesters produced according to the process of the invention can be linear or branched depending on the monomers selected and on the reaction conditions and can be obtained with functional groups (for example hydroxyl) for further conversion of the same into other polymers, for example polyurethanes.

According to a preferred embodiment, polyesters produced through the process of the invention can comprise or consist of repeating units of one diacid and one diol; one diacid and one polyol; one diacid, one diol and one polyol; one diacid, one diol and one hydroxy acid; one diacid, one polyol and one hydroxy acid; one diacid, one polyol and one hydroxy acid; one diacid, one diol, one polyol and one hydroxy acid; one diacid, one diol and one hydroxy amine, or other possible combinations of monomers, for example combinations wherein the diacid is substituted with a methyl ester or ethyl ester derivative thereof.

Preferred combinations of monomers are adipic acid/butanediol/ethanolamine, dimethyl adipate/ 1,4-butanediol, adipic acid/methyl ethanolamine, ethanolamine/adipic acid, diethanolamine/adipic acid, ethanolamine/dimethyl adipate, N-methyl ethanolamine/dimethyl adipate, diethanolamine/dimethyl adipate, adipic acid/glycerol, adipic acid/1,4-butanediol, adipic acid/diethylene glycol, adipic acid/diethylene glycol/glycerol, adipic acid/diethylene glycol/ trimethylol propane, diethylene glycol/adipic acid/dimethylol propane, adipic acid/ 1,6-hexanediol.

Reactive carboxylic groups and reactive amine and hydroxyl groups of the reagents are generally present essentially in the same number. However, it is also possible to perform the polymerization reaction in stoichiometric defect, even if this usually gives rise to a product having a lower number average molecular weight in comparison with that achievable when reagents are employed in equimolar amounts. In some cases, the relative amounts of the reagents can also be adjusted so to have a terminal acid, hydroxyl, thiol or amine functional group in the final polyesters to allow further conversion or use of the same in other products. For example, polyesters having acid terminal functional groups are useful in a variety of coating compositions while polyesters having hydroxyl terminal functional groups can be used as reagents for the production of polyurethanes.

In the process according to the invention, the enzyme can be in a free or immobilized form where the separation of the enzyme in an intermediate step of the polymerization is not provided for (single step process) while the enzyme needs to be immobilized where the separation of the enzyme in an intermediate step of the polymerization is provided for (two step process).

The enzyme immobilization is preferably carried out in a covalent way and optionally on an inert support (polymeric support), that is to say through the formation of intermolecular covalent bonds between enzymatic molecules and/or covalent bonds between enzyme and support.

For example, the enzyme immobilization on the polymeric support can occur through covalent bonds between respective suitable functional groups (optionally introducing suitable functional groups on the support) or using a bifunctional reagent (for example, glutaraldehyde) able to form a net of covalent bonds (cross-links) between the enzymatic molecules, which keep such enzymatic molecules adherent to the solid support. Alternatively the enzymatic molecules can be bound together through a bifunctional reagent able to create covalent bonds (cross-links) to form a solid form of the biocatalyst in the absence of a solid support.

Preferably, in the process of the invention an enzyme covalently immobilized on a polymeric support is used.

In the cases wherein an enzyme immobilized on a support is used, it should be noted that the enzyme can be readily removed from the reaction mixture without employing complicated purification procedures, for example by filtration or centrifugal separation. Advantageously, the enzyme recovered from the reaction medium can be recycled for use in a further polymerization cycle, as already indicated previously.

In the process according to the invention, the enzyme can be present in the reaction mixture until completion of the reaction itself. Alternatively, the enzyme can be immobilized and removed from the reaction mixture after completion of the initial oligomerization step for example by filtration or centrifugal separation. In the latter case, the subsequent polymerization step in the absence of the immobilized enzyme is carried out preferably with continuous removal of water or other reaction byproducts (for example, alcohols) so as to lead the polymerization reaction towards the formation of the products.

Additionally, the subsequent polymerization step in the absence of the immobilized enzyme can be advantageously accelerated by increasing the temperature (in comparison with that employed in the presence of the enzyme) at least to 50°C, preferably between 90°C and 300°C, most preferably between 90°C and 180°C.

Preferably, in the process according to the invention, the enzyme consists of a hydrolase able to catalyze polyesterification reactions and, most preferably, of a lipase.

Preferably, the enzyme used in the process according to the invention consists of a lipase derived from *Candida antarctica* (hereunder also called CALB) in a free form or preferably immobilized on an inert support.

Such enzyme is commercially available, with the lipase that can be obtained by techniques of recombinant DNA or by extraction from an organism that contains it.

According to a particularly preferred embodiment, the enzyme used in the process according to the invention consists of CALB covalently immobilized on a middle hydrophobic support, in particular an acrylic resin functionalized with epoxy groups. In this regard, particularly preferred is the employment of materials consisting of CALB immobilized on a support obtained according to the immobilization techniques described in the Italian Patent Application number PD2010A000392 filed December 23, 2010 by Sprin S.p.A. and Università degli Studi di Trieste. Such application is here incorporated as a reference both relating to materials consisting of CALB immobilized on the support described therein, and relating to the nature of the supports employed and the corresponding enzyme immobilization techniques on such supports.

The employment of an enzyme covalently immobilized on a support, in particular of materials consisting of CALB immobilized on a support as described in said Patent Application number PD2010A000392, results to be particularly advantageous in the process according to the invention since it increases the stability of the biocatalyst avoiding mechanical damage, for example, due to the action of the bladed rotor, during the turbulent flow of the thin layer of reaction mixture along the wall kept at a predetermined temperature. In such way, it was advantageously found that separation (leaching) of the enzyme from the support with consequent release (and loss) of it in the reaction mixture is prevented.

The amount of enzyme employed in the process according to the invention can be determined according to the needs and in particular according to considerations of expressed catalytic activity, conversion and speed of reaction, as well as reduction of production costs. By using CALB in the process according to the invention, it is preferable to employ 0.1% to 20% by weight of the biocatalyst (meant both as enzyme in the free form and as amount of immobilized enzyme) on the total weight of the monomers.

The process according to the invention can be carried out at atmospheric pressure or under reduced pressure. It should be noted anyway that, according to the invention, the fact of making the reaction mixture flow in the form of a thin, turbulent and dynamic layer in contact with a heated wall already allows an efficient removal of water and/or other reaction byproducts (for example alcohols) from the reaction mixture itself. Anyway, in some cases, it can be convenient to remove water and/or other reaction byproducts also in a forced manner, in particular reducing the pressure, at which the reaction is carried out. In such case, the pressure can be reduced to be equal to or lower than 700 mmHg (93.3 kPa), preferably comprised between 650 mmHg (86.6 kPa) and 700 mmHg (93.3 kPa). The application of reduced pressure can be carried out both in the oligomerization step and in the subsequent polymerization step of the oligomers in the final product.

The process according to the invention allows the production of high weight average molecular weight (Mw) polyesters and polyester amides. In particular, such weight average molecular weight has a minimum of 200 Da, and is preferably comprised between 1500 Da and 15000 Da or higher.

The weight average molecular weight can be determined according to methods per se conventional, such as for example gel permeation chromatography (GPC), nuclear magnetic resonance (NMR) and viscosimetry.

Additionally, polyesters and polyester amides obtained according to the process of the invention advantageously have a low polydispersity index (PDI) meant as the ratio between weight average molecular weight (Mw) and number average molecular weight (Mn). Such index can be determined with methods per se conventional, and is advantageously lower than 1.5, preferably between 1.1 and 1.4.

The process according to the invention can be carried out in the presence of a solvent or preferably in the absence of a solvent. The term "solvent" should mean any liquid that is essentially inert in the reaction conditions of the process according to the invention.

The starting monomers, the biocatalyst and the optional solvent and/or additives as described above are preferably mixed, in one or several mixing steps to constitute the reaction mixture, before the treatment of the mixture in the reaction conditions of the present invention (thin and turbulent layer made move forward along a wall at a predetermined temperature), for example in a turbo-reactor as above.

However, it is also possible to separately introduce the reagents or a part of them (for example additives) and the optional solvent in the reaction vessel (for example a turbo-reactor as above) to form the reaction mixture *in situ.*

As it can be noted from what exposed above, the process according to the invention achieves a high efficiency of conversion by increasing the reaction kinetics by virtue of improved mass and thermal transfer and improved diffusive processes.

Additionally, the process allows to markedly reduce production times and costs, also thanks to the fact that the biocatalyst can be recycled and employed for different production cycles, in which it maintains a suitable catalytic efficiency. This is illustrated in particular in example 2, reported hereunder in the present description.

Hence, the process of the invention is suitable for application on an industrial level. To this regard, it should be additionally noted that the process of the invention involves reduced energetic expenditure and allows continuous processing, with consequent increase of the hourly productivity and time optimization.

Further advantages and characteristics of the process according to the present invention will be even more apparent from the description of an embodiment thereof, provided hereunder by way of illustration and not of limitation, with reference to the drawings attached herein.

### Short description of the figures

In the drawings:
- figure 1 illustrates in a schematic way an apparatus for the execution of the method according to the invention;
- figure 2 shows some scanning electron micrographs with 100 x enlargement of a sample of biocatalyst recovered at the end of 3 cycles of conversion carried out according to the process of the invention.

### Detailed description

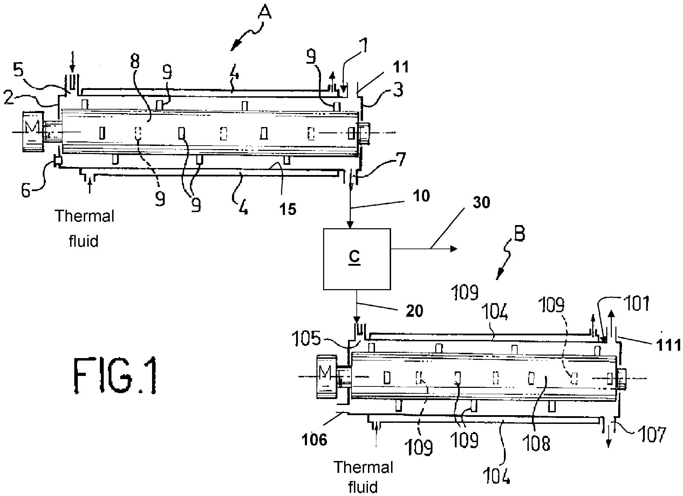

With reference to figure 1, the apparatus used for the production of polyesters according to the process of the invention comprises a first turbo-reactor A, a second turbo-reactor B and a separator C in fluid-flow communication between them.

In particular, the separator C is interposed between turbo-reactors A and B and in fluid-flow communication with them, through the respective flow lines 10 and 20.

The term "flow line" should generally mean a connection pipe or tubing, conventional as such, along which suitable functional devices (for example, pumps) can be placed for the transport of material from one unit to a following unit of the apparatus according to the invention.

In the present example, the first turbo-reactor A is used for the conversion of reagents (a monomer or a combination of monomers as defined above) into oligomers (oligomerization step) in the presence of a biocatalyst in the reaction conditions of the process according to the invention.

The second turbo-reactor B, instead, is used for the conversion of a mixture of oligomers coming out of the first turbo-reactor A, freed of the biocatalyst by the separator C, into polyesters in the reaction conditions of the process of the invention.

Turbo-reactors A and B are preferably manufactured by the firm VOMM Impianti e Processi, Rozzano, Milan (Italy).

More in particular, turbo-reactor A essentially comprises a tubular cylindrical body 1 closed at the opposite ends by the bottoms 2, 3 and provided with a coaxial heating or cooling jacket 4 (depending on the needs), inside which a thermal fluid is destined to flow, e.g. diathermic oil, steam or warm or overheated water or a cooling fluid, to keep the internal wall of the body 1 at a predetermined temperature.

The tubular body 1 is provided with at least one first input opening 5 (in figure 1 one single input opening 5 is shown, for the sake of simplicity), to feed into the tubular body 1 a reaction mixture comprising at least one monomer, the biocatalyst and the optional solvent and at least a second input opening 6 (in figure 1 one single input opening 6 is shown, for the sake of simplicity) for an inert gas and/or additives, as defined previously, to be added to the reaction mixture entering the turbo-reactor B through the at least one first opening 5.

For technical incidental reasons the turboreactor A can obviously have more than one input opening, for example to separately feed reagents (at least one monomer), biocatalyst (preferably pre-mixed with the at least one monomer), optional solvent, inert gas and/or optional additives to form the reaction mixture directly *in situ* inside the turb-reactor B. In such case, it is preferable that the biocatalyst is pre-mixed with the at least one reagent or with the optional solvent.

Inside the tubular body 1, a bladed rotor 8 is rotably supported. Blades 9 of this rotor are arranged in a helicoidal way and oriented in a way to centrifuge and simultaneously move the reaction mixture undergoing the treatment towards the exit in the form of a thin, turbulent and dynamic layer in contact with a heated or cooled internal wall 15 of the tubular body 1. A motor M provides the rotor 8 with rotation at a speed comprised between 150 and 700 rpm, preferably 350 rpm.

The tubular body 1 is additionally provided with a first output opening 7 for the reaction product formed in the first turbo-reactor A (mixture of oligomers + biocatalyst) and a second output opening 11 (vent) for the optional inert gas introduced in the turbo-reactor A and/or for vapors formed during the treatment of the reaction mixture, mainly water and/or other reaction byproducts that are removed from it in their vapor state.

For technical incidental reasons turbo-reactor A can obviously have more than one output opening for the reaction product and/or as a vent for vapors.

In some cases, the second output opening 11 can be connected with an apparatus to create a vacuum, conventional as such, so as to apply a reduced pressure inside the turbo-reactor A that favors the removal of water and/or other reaction byproducts, moving the balance of the oligomerization reaction towards the formation of the products.

The second turbo-reactor B is fully similar to the first turbo-reactor A and as a consequence it is not further described. The parts of the turbo-reactor B that are structurally and/or functionally equivalent to the corresponding parts of the turbo-reactor A are indicated in figure 1 with the same reference numbers used for the first turbo-reactor A but increased by 100.

According to the process of the invention, the reagents (at least one monomer) and the biocatalyst are mixed together in a blender, preferably in a continuous way, to form a mixture. The biocatalyst can be both "fresh" and recycled, coming from a production cycle, as long as it maintains a suitable catalytic activity, or a mixture of both.

The blender can be any apparatus conventional as such provided with a suitable stirrer and optionally heated to allow suitable mixing of reagents and biocatalyst until a reaction mixture as homogeneous as possible and suitable to be transferred, for example with the aid of a pump, into the following unit of the apparatus according to the invention is formed.

Optionally, if necessary or proper, the biocatalyst can be pre-mixed with or suspended on one of the reagents before being fed into said blender.

The reaction mixture coming out of the blender is continuously fed into the first turbo-reactor A through the first input opening 5.

Simultaneously, or in a subsequent time, a flow of additives, as defined above or a flow of an inert gas (for example nitrogen) is continuously and in co-current fed into the turbo-reactor A through the second input opening 6. Obviously, where the use of additives and an inert gas were not necessary, the second opening 6 could be omitted.

In the turbo-reactor A, the reaction mixture comprising at least one monomer and the biocatalyst and optionally provided with at least one additive undergoes the mechanical action of the blades 9 of the rotor 8 and as a consequence it is split and centrifuged, since its entrance into the turbo-reactor A, against the internal wall 15 to form a thin and turbulent layer that is simultaneously directed towards the output opening 7 thanks to the helicoidal inclination of said blades 9.

Advantageously, the internal wall 15 is kept at an essentially constant temperature, preferably comprised between 45°C and 130°C by means of the thermal fluid flowing through the jacket 4.

Thanks to the formation of a tubular, thin, dynamic, and turbulent layer by means of the action of the bladed rotor set in rotation at high speed, and of the controlled temperature of the wall in contact with such layer, optimal conditions of mass and thermal transfer are established, to the whole advantage of the efficiency and yield of conversion into oligomers or polymers.

More in particular, it has been surprisingly found that the mechanical action of splitting and centrifugation of the reaction mixture operated by the bladed rotor allows optimal conditions of contact between the biocatalyst and the mass to catalyze, as well as it exerts optimal shear forces on the oligomer or polymer in the making without determining a substantial damage of the biocatalyst by mechanical action, in particular where the biocatalyst is immobilized on a support (preferably in a covalent way).

At the same time, the fact that the thin layer of mixture is made move forward in a turbulent flow and in continuous contact with a part at a constant temperature, advantageously allows to effectively and continuously remove the reaction byproducts (mainly water, but also for example, alcohols) in the form of vapors from the reaction mixture thereby promoting the reaction of polycondensation to form oligomers or polymers.

Advantageously, water and possible other byproducts in the state of vapors are removed from the turbo-reactor A through the second output opening 11 that is preferably connected with an apparatus (not shown) to create a vacuum inside the turbo-reactor A.

It should be additionally noted that in the process conditions according to the invention and with the continuous removal of water from the reaction mixture (in the form of steam) there is also the advantageous effect of being able to exploit the latent heat of evaporation of water and the optionally negative enthalpy of the reaction of polycondensation. This advantageously allows to keep the temperature, to which the polymeric chain in the making is subjected, at values lower than the predetermined temperature of the internal part 15 of the turbo-reactor B, hence remaining in a safe and continuously manageable range of temperatures.

After a residence time in the turbo-reactor A comprised between 6 and 30 minutes, a product consisting of a mixture comprising oligomers and biocatalyst is continuously discharged through the first output opening 7.

Optionally, if desired, the mixture comprising oligomers and biocatalyst coming out of the turbo-reactor A can be recycled one or several times in the same turbo-reactor A, through the first input opening 5 for further cycles of conversion so as to obtain a final product (oligomers) in a desired range of chain length.

Alternatively, the mixture comprising oligomers and biocatalyst coming out of the turbo-reactor A is fed into the separator C through the flow line 10. The separator C is conventional as such and can be for example a filtration unit or a centrifugal separator that provides the separation of the biocatalyst from the mixture of oligomers.

The biocatalyst separated from the mixture of oligomers comes out of the separator C through the flow line 30 and can be advantageously recovered for a new polycondensation cycle so as to optimize the efficiency of the process of the invention and reduce related costs.

The mixture comprising oligomers and free of biocatalyst coming out of the separator C through the flow line 30, instead, is fed into the second turbo-reactor B through its first opening 105.

Such mixture is treated in the second turbo-reactor B in a manner analogous to that described above referring to the first turbo-reactor A, to obtain the conversion of the oligomers into the final polyesters obtaining the same advantages described above.

In the turbo-reactor B, however, being the mixture subjected to the treatment free of biocatalyst, the temperature of the internal part 115 can be properly increased to values higher than those of the wall 15 of the first turbo-reactor A. Such temperature is preferably comprised between 90°C and 180°C.

The residence times of the reaction mixture in the turbo-reactor B instead, are preferably comprised between 5 and 20 minutes.

At the end of the treatment, the reaction product (mixture of polyesters) is discharged from the turbo-reactor B through the second output opening 107 and recovered for further treatments that can comprise, for example, a purification step, if necessary and/or the conversion into other products (for example polyurethanes).

Hereunder, some examples of realization of the process according to the invention are reported, by way of indication and not of limitation.

### EXAMPLE 1

In the present example a polycondensation of a diol and a diacid, achieved according to the process of the invention is illustrated.

The diol consists of 1,4-butanediol, the diacid consists of adipic acid and the biocatalyst (enzyme) consists of a catalytic material provided by the firm Sprin S.p.A., Trieste (Italy) and consisting of CALB immobilized in a covalent way on an organic porous acrylic polymer functionalized with epoxy groups.

Such catalytic material is obtained according to the methods described in the cited Italian Patent Application number PD2010A000392 (in particular according to example 18). In this example, a catalytic material having catalytic activity of 300 U/g was used.

Catalytic activity is expressed as hydrolytic activity towards the substrate tributyrin in the following conditions:
2 ml of 0.1M Kpi buffer (potassium hydrogen/dihydrogen phosphate), pH 7.0 are added to 30 ml of substrate in the form of an emulsion (100 ml of emulsion are prepared using 5 ml of tributyrin, 17 ml of a 0.3M NaCl solution, 6% gum arabic in glycerol (54% v/v) and 78 ml of ultrapure water). After equilibration of the pH, 30-50 mg of immobilized enzymatic preparation are added and the released butyric acid is titrated with a 0.1M NaOH solution. The hydrolytic activity is determined according to the following assay that employs a pH-stat system (Mettler-Toledo T50). One unit corresponds to the amount of enzyme that hydrolyzes 1 micromole of tributyrin in one minute at 30°C.

Using the apparatus schematically described above and following the method of the invention, a flow of 32 kg/h of adipic acid is fed through a first input opening 5 and simultaneously a flow of 26 kg/h of a mixture of biocatalyst and 1,4-butanediol (mixed together in a conventional ribbon blender in such proportions to obtain 21% biocatalyst by weight on the weight of the mixture biocatalyst + diol) is fed through another first input opening 5 in the first turbo-reactor A. The components, which the turbo-reactor is fed with and which are mixed therein, have a molar ratio diacid/diol equal to about 1:1.05 and a content of biocatalyst equal to about 9% by weight on the weight of the overall mixture comprised of the biocatalyst plus the reagents (monomers).

Such turbo-reactor A has a diameter of 350 mm, operates at a throughput of 58 kg/h and has no second input opening 6 for additives and/or inert gas.

The internal wall 15 temperature is kept at about 86°C, which involves an actual temperature of the reaction mixture of about 60°C. The rotation speed of the bladed rotor 8 is kept constant at 350 rpm. In this specific case the reaction is carried out at atmospheric pressure.

After a mean residence time in the turbo-reactor A of 12 minutes, a flow consisting of a mixture comprising oligomers and biocatalyst is continuously discharged through the first output opening 7, which is recovered and recycled inside the turbo-reactor A for two further and subsequent cycles of treatment in the same conditions indicated above but with a mean residence time inside the turbo-reactor A of about 16 minutes for each cycle.

At the end of the three overall cycles of treatment in the turbo-reactor A, the resulting mixture comprising oligomers and enzyme coming out of the second output opening 7 is transferred into the separator C, in this specific case a filtration unit.

Inside the separator C, the mixture coming from the turbo-reactor A is forced through sieves having mean size of the pores comprised between 80 µm and 100 µm, obtaining the separation of the biocatalyst from the mixture of oligomers.

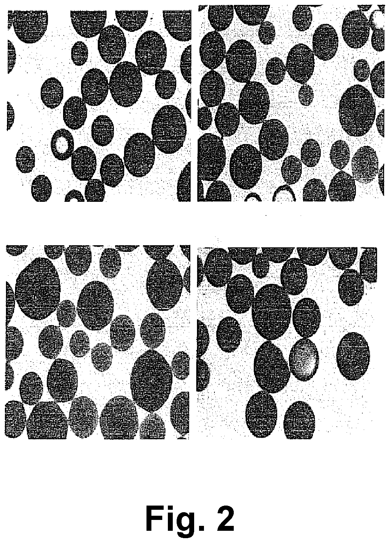

The biocatalyst is recovered and can be recycled for further cycles of treatment inside the first turbo-reactor A. Advantageously, as it can be noted from figure 2, the biocatalyst particles recovered after said three cycles in the turbo-reactor A show an essentially regular profile (not different from the original one) indicating then that the biocatalyst is not significantly damaged by the mechanical action in the reaction conditions of the present invention.

The filtrate coming out of the separator C (mixture of oligomers essentially free of biocatalyst), instead, is fed into the second turbo-reactor B, essentially identical to the first turbo-reactor A, through the input opening 105 and there treated to convert the oligomers into the polymeric final product.

The operating conditions of the second turbo-reactor are the following:
- temperature of the wall 115: about 110°C corresponding to an actual temperature of the reaction mixture about 90°C;
- bladed rotor 108 speed: 350 rpm;
- mean residence time: 10 minutes.

At the end of the treatment in the second turbo-reactor, a polymeric product (polyesters) is discharged in the form of a substantially transparent liquid.

Results of the polycondensation process are reported in the following table 1.

**Table 1**

| **Sample** | **Biocatalyst*** | **Adipic acid // 1,4-butanediol** | **Reaction conditions** | **Mn and (PDI)** |
|---|---|---|---|---|
| After the 1^{st} cycle in turboreactor A | 10.3% | 32 kg/h // 26 kg/h** | 60°C, 350 rpm, 12 minutes | 400*** |
| After the 2nd cycle in turboreactor A | 10.3% | Not detected | 60°C, 350 rpm, 16 minutes | 1300 (1.45)**** |
| After the 3^{rd} cycle in turboreactor A | 10.3% | Not detected | 60°C, 350 rpm, 16 minutes | 1800 (1.33)**** |
| After cycle in turbo-reactor B | Not present | Not detected | 90°C, 350 rpm, 10 minutes | 2900 (1.34)**** |

| | | | | |
|---|---|---|---|---|
| * by weight on the overall weight of the monomers (adipic acid/1,4-butanediol) ** 1,4-butanediol and biocatalyst mixture *** Mn estimated by determining the ratio of the signal (peak) at δ 4.08 to the signal at δ 3.53 of the ¹H-NMR spectrum of the raw product i.e. the increase of the ratio of the signal at δ 4.08 (t, 2H, -CH₂OC(O)), corresponding to the formation of the ester, to the signal at δ 3.53 (t, 2H, -CH₂-CH₂-OH) that is, instead, attributable to "free" 1,4-butanediol, i.e. not polymerized ****determined by gel permeation chromatography using a high pressure chromatography system connected with a GPC Waters 150C detector, "evaporative light scattering" detector and a PolyPore column 7.5 mm long, eluent THF (flow rate 1 ml/min at 30°C). Nine polystyrene samples were used as standards (Mn 680, 1060, 1100, 2450, 5050, 7000, 11600, 22000, 66000). For each analysis, about 7 mg of sample were dissolved in THF and analyzed without pretreatment. From what illustrated above, it can be noted that a polymeric product is obtained, which displays a high value of Mn and low PDI. | | | | |

### EXAMPLE 2

In the present example a polycondensation of a diol and a diester of a diacid, achieved according to the process of the invention is illustrated.

The diol consists of 1,4-butanediol, the diester of a diacid consists of diethyl adipate and the biocatalyst (enzyme) consists of the same catalytic material reported in example 1.

Using the apparatus schematically described above and following the method of the invention, a flow of 44 kg/h of diethyl adipate is fed through a first input opening 5 and, simultaneously, a flow of 21.7 kg/h of a mixture of biocatalyst and 1,4-butanediol (mixed together in a conventional ribbon blender - 20.6 kg/h of diethyl adipate and 1.1 kg/h of biocatalyst) is fed through another first input opening 5 into the first turbo-reactor A. The components, which the turbo-reactor is fed with and which are mixed therein have a molar ratio diacid/diol equal to about 1:1.05 and a content of biocatalyst equal to about 1.7% by weight on the weight of the overall mixture comprised of biocatalyst plus the reagents. Such turbo-reactor A has a diameter of 350 mm at a throughput of 65.7 kg/h and has no second input opening 6 for additives and/or inert gas.

The internal wall 15 temperature is kept at about 70°C, which means an actual temperature of the reaction mixture of about 50°C. The rotation speed of the bladed rotor 8 is kept constant at 350 rpm. In this specific case the reaction is carried out at atmospheric pressure.

After a mean residence time in the turbo-reactor A of 12 minutes, a flow consisting of a mixture comprising oligomers and biocatalyst is continuously discharged through the output opening 7, which is recovered and transferred into the separator C, in this specific case a filtration unit.

Inside the separator C, the mixture coming from the turbo-reactor A is forced through sieves having mean size of the pores comprised between 80 µm and 100 µm, obtaining the separation of the biocatalyst from the mixture of oligomers. Oligomers are then analyzed according to the procedure described in example 1 and result to have Mn = 2000 and PD = 1.35.

The biocatalyst recovered in the separator C is recycled for further 8 cycles of treatment inside the first turbo-reactor A according to the procedure described here above. After each reaction cycle a sample equal to about 30-50 mg of supported enzyme is taken, and its enzymatic activity is evaluated according to the method described in example 1.

The biocatalyst samples indicated in all the cases an activity at least equal to 85% of that expressed by the original biocatalyst, demonstrating the recyclability of the biocatalyst and the preservation of its activity.

### EXAMPLE 3

In the present example a polycondensation of a diol and a diester of a diacid achieved according to the process of the invention is illustrated.

The diol consists of 1,4-butanediol, the diester of a diacid consists of diethyl adipate and the biocatalyst (enzyme) consists of the commercial enzymatic preparation Novozym 435 comprised of lipase from *Candida antarctica* adsorbed on an acrylic solid support. The polycondensation was performed according to the procedure described in example 2.

Inside the separator C, the mixture coming from the turbo-reactor A was forced through sieves having mean size of the pores comprised between 80 µm and 100 µm, obtaining the separation of the biocatalyst from the mixture of oligomers. Oligomers were then analyzed according to the procedure described in example 1 and resulted to have Mn=2400 and PD=1.41.

The biocatalyst recovered in the separator C is recycled for further 8 cycles of treatment inside the first turbo-reactor A according to the procedure described here above. After each reaction cycle a sample equal to about 30-50 mg of supported enzyme is taken, and its enzymatic activity evaluated according to the method described in example 1.

After 1 cycle, the catalyst results to have retained about 60% of its activity and after 3 cycles of polycondensation the catalyst retains 40% of its original activity, demonstrating that the enzymatic preparation adsorbed on a solid support is not suitable for use in the process described above, since the enzymatic molecules detach from the support as described in the paper C. Korupp; R. Weberskirch; J.J. Müller; A. Liese; L. Hilterhaus, Organic Process Research & Development 2010, 14, 1118-1124.

## Claims

1. Process for the production of polyesters through polymerization or polycondensation catalyzed by enzyme of at least one monomer **characterized in that** it comprises the step of running a mixture comprising said at least one monomer and said enzyme in the form of a thin, turbulent and dynamic layer in contact with a wall kept at a predetermined temperature.

2. Process according to claim 1, comprising the following steps:
- preparing a mixture comprising at least one monomer and an enzyme,
- running at least once said mixture in the form of a thin, turbulent and dynamic layer in contact with a wall kept at a predetermined temperature, obtaining a polymeric product comprising polyesters.

3. Process according to claim 1, comprising the following steps:
- preparing a mixture comprising at least one monomer and an immobilized enzyme,
- running at least once said mixture in the form of a thin, turbulent and dynamic layer in contact with a wall kept at a predetermined temperature, obtaining a mixture comprising oligomers,
- essentially separating said immobilized enzyme from said mixture comprising oligomers,
- running at least once said mixture comprising oligomers and essentially free of said immobilized enzyme in the form of a thin, turbulent and dynamic layer in contact with a wall kept at a predetermined temperature obtaining a polymeric product comprising polyesters.

4. Process according to any one of preceding claims, wherein said wall is kept at a temperature at least of 35°C, preferably between 45°C and 130°C, in the case of treatment of said mixture containing said at least one monomer and said enzyme (optionally immobilized), or at a temperature at least of 50°C, preferably between 90°C and 300°C, in the case of treatment of said mixture containing oligomers and free of said immobilized enzyme.

5. Process according to claim 1 or claim 2 comprising the steps of:
- providing a turboreactor (A) comprising a tubular cylindrical body (1), provided with a heating or cooling jacket (4), closed at the opposite ends by bottoms (3, 4), provided with at least one input opening (5, 6) and at least one output opening (7, 11), and inside which a coaxial bladed rotor (8) is rotably supported,
- feeding, through said at least one input opening (5), a continuous flow of a mixture comprising at least one monomer and an enzyme into said turbo-reactor (A), or feeding, through respective input openings (5), a continuous flow comprising at least one monomer and a separated continuous flow comprising an enzyme and optionally at least one monomer into said turbo-reactor (A), so as to form said mixture inside of it, the temperature of the wall of said turbo-reactor (A) being at least 35°C, preferably between 45°C and 130°C,
- subjecting at least once said mixture to the mechanical action of the bladed rotor (8) set in rotation at a speed of at least 150 rpm, with the consequent centrifugation of said mixture against the heated wall and formation of a thin, tubular and dynamic layer, wherein said mixture is mechanically kept in a condition of turbulence by the blades (9) of said bladed rotor (8),
- making said thin, turbulent and dynamic layer move forward towards said at least one output opening (7) of the turbo-reactor (A),
- continuously discharging a flow of a mixture comprising oligomers and/or polyesters from said at least one output opening (7) of said turbo-reactor,
- optionally separating said enzyme from said mixture comprising polyesters.

6. Process according to claim 3 comprising the steps of:
- providing a first turbo-reactor (A) and a second turbo-reactor (B), each comprising a tubular cylindrical body (1; 101), provided with a heating or cooling jacket (4; 104), closed at the opposite ends by bottoms (3, 4; 103 ,104), provided with at least one input opening (5, 6; 105, 106) and at least one output opening (7, 11; 107, 111) and inside which a coaxial bladed rotor (8; 108) is rotably supported,
- feeding, through said at least one input opening (5), a continuous flow of a mixture comprising at least one monomer and an immobilized enzyme into said first turbo-reactor (A), or feeding, through respective input openings (5), a continuous flow comprising at least one monomer and a separated continuous flow comprising an immobilized enzyme and optionally at least one monomer into said first turbo-reactor (A), so as to form said mixture inside of it, the temperature of the wall of said turbo-reactor (A) being at least 35°C, preferably between 45°C and 130°C,
- subjecting at least once said mixture to the mechanical action of the bladed rotor (8) set in rotation at a speed of at least 150 rpm, with the consequent centrifugation of said mixture against the heated wall and formation of a thin, tubular and dynamic layer, wherein said mixture is mechanically kept in a condition of turbulence by the blades (9) of said bladed rotor (8),
- making said thin, turbulent and dynamic layer move forward towards said at least one output opening (7) of the turbo-reactor (A),
- continuously discharging a flow of a mixture comprising oligomers from said at least one output opening (7) of said first turboreactor (A),
- separating said immobilized enzyme from said mixture comprising oligomers, obtaining a mixture of oligomers essentially free of said immobilized enzyme,
- feeding, through said at least one input opening (105), a continuous flow of said mixture of oligomers essentially free of said immobilized enzyme in said second turbo-reactor (B), which temperature of the wall is at least 50°C, preferably between 90°C and 300°C,
- subjecting said mixture of oligomers essentially free of said immobilized enzyme to the mechanical action of the bladed rotor (108) of said second turbo-reactor (B) set in rotation at a speed of at least 150 rpm, with the consequent centrifugation of said mixture against the heated wall and formation of a thin, turbulent and dynamic layer, wherein said mixture is mechanically kept in a condition of high turbulence by the blades (109) of said bladed rotor (108),
- making said thin, turbulent and dynamic layer move forward towards said at least one output opening (107) of said second turbo-reactor (B),
- continuously discharging a flow of a mixture of polyesters from said at least one output opening (107) of said second turbo-reactor (B).

7. Process according to claim 5 or 6, wherein the rotation speed of the bladed rotor (8; 108) of said turbo-reactor (A), or of said first turbo-reactor (A) and second turboreactor (B) is comprised between 150 and 700 rpm.

8. Process according to any one of claims 5 to 7, wherein the residence time of the mixture comprising the at least one monomer and an enzyme or of the mixture of oligomers essentially free of enzyme in the respective turbo-reactor (A; B) is comprised between 5 minutes and 30 minutes.

9. Process according to any one of claims 5 to 8, further comprising the step of feeding into said turbo-reactor (A) or into one or both of said first turbo-reactor (A) and second turbo-reactor (B), through said at least one input opening (6; 106), a continuous flow of an inert gas and/or additives, in co-current with said flow of mixture comprising the at least one monomer and an enzyme and/or with said flow of oligomers free of enzyme.

10. Process according to any one of claims 5 to 9, wherein in said turbo-reactor (A) or in one or both of said first turbo-reactor (A) and second turbo-reactor (B) a reduced pressure is applied equal to or lower than 700 mmHg (93.3 kPa), preferably comprised between 650 mmHg (86.6 kPa) and 700 mmHg (93.3 kPa).

11. Process according to claim 3 or to any one of claims 6 to 10 wherein said separation step of the immobilized enzyme is carried out by filtration or centrifugal separation.

12. Process according to any one of preceding claims, wherein said at least one monomer consists of a diol and a diacid or a derivative thereof, preferably a diester or a lactone.

13. Process according to any one of preceding claims, wherein said enzyme consists of a hydrolase, preferably a lipase, and more preferably a lipase derived from *Candida antarctica* (CALB) in a free or immobilized form.

14. Process according to claim 13, wherein said enzyme consists of CALB immobilized in a covalent way, preferably on an inert support.

15. Process according to any one of preceding claims, wherein the amount of said enzyme in a free or immobilized form is comprised between 0.1% and 20% by weight on the overall weight of said at least one monomer.
